# EUROPÄISCHE PATENTSCHRIFT

(11) **EP 2 732 392 B1**
(45) Veröffentlichungstag und Bekanntmachung des Hinweises auf die Patenterteilung: **29.04.2020**
(21) Anmeldenummer: 12734815.9
(22) Anmeldetag: 07.07.2012
(51) Int. Cl.: G16H 40/67

(54) **VERFAHREN UND VORRICHTUNG ZUR ENTFERNTEN ÜBERWACHUNG UND STEUERUNG VON MEDIZINISCHEN FLUIDMANAGEMENTGERÄTEN**
METHOD AND DEVICE FOR REMOTE MONITORING AND CONTROLLING OF MEDICAL FLUID MANAGEMENT DEVICES
PROCÉDÉ ET DISPOSITIF DE SURVEILLANCE ET COMMANDE À DISTANCE D'APPAREILS MÉDICAUX DE GESTION DE FLUIDES

(30) Priorität: 15.07.2011 DE 102011107795; 15.07.2011 US 201161457947 P
(43) Veröffentlichungstag der Anmeldung: 21.05.2014
(73) Patentinhaber: Fresenius Medical Care Deutschland GmbH, 61352 Bad Homburg (DE)
(72) Erfinder: BRAND, Georg, 97076 Würzburg (DE); HAUCK, Daniel, 97529 Sulzheim (DE); KLÖFFEL, Peter, 97720 Nüdlingen (DE); STAHL, Thomas, 97839 Esselbach (DE)
(74) Vertreter: Dreyhsig, Jörg
(86) Internationale Anmeldenummer: PCT/EP2012/002872
(87) Internationale Veröffentlichungsnummer: WO 2013/010642

(56) Entgegenhaltungen:
- WO-A1-2007/126360
- WO-A1-2009/122270
- US-A- 5 472 614
- US-A1- 2010 137 693
- US-A1- 2011 066 006
- US-B2- 7 044 927
- Pennsylvania Patient Safety Advisory: "Hemodialysis Administration", , 1 January 2010 (2010-01-01), XP055566182, Retrieved from the Internet: URL:http://patientsafety.pa.gov/ADVISORIES /documents/201009_87.pdf [retrieved on 2019-03-07]

## Beschreibung

Die Erfindung betrifft ein Verfahren und eine Vorrichtung zur entfernten Überwachung und Steuerung von medizinischen Fluidmanagementgeräten.

Unter medizinischen Fluidmanagementgeräten werden hierbei insbesondere Geräte zur Leitung, Behandlung und/oder Verteilung von Flüssigkeiten und/oder Gasen verstanden, bei denen über eine Fluidleitung Fluid zwischen einem Patienten und einer Fluidbehandlungskomponente und/oder einer Fluidquelle transportiert werden.

Unter Fluidmanagementgeräten werden insbesondere auch Fluidbehandlungsgeräte wie Blutbehandlungsgeräte verstanden, bei denen ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben wird. Beispiele für solche Blutbehandlungsgeräte sind insbesondere Hämodialysegeräte. Ein solches Blutbehandlungsgerät ist Gegenstand der DE 198 49 787 C1 der Anmelderin.

Die Dialyse ist ein Verfahren zur Blutreinigung von Patienten mit akuter oder chronischer Niereninsuffizienz. Grundsätzlich unterscheidet man hierbei Verfahren mit einem extrakorporalen Blutkreislauf, wie die Hämodialyse, die Hämofiltration oder die Hämodiafiltration (im Folgenden unter dem Begriff Hämodialyse zusammengefasst) und der Peritonealdialyse, die keinen extrakorporalen Blutkreislauf aufweist.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration der Dialysierflüssigkeit entspricht der Konzentration des Blutes eines Gesunden. Während der Behandlung werden das Blut des Patienten und die Dialysierflüssigkeit an beiden Seiten der Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Wird an der Dialysemembran ein Druckgradient von der Blutseite zur Dialysatseite aufgebaut, beispielsweise durch eine Pumpe, die flussabwärts des Dialysefilters auf der Dialysatseite Dialysat aus dem Dialysatkreislauf entzieht, tritt Wasser aus dem Patientenblut über die Dialysemembran in den Dialysatkreislauf über. Dieser Vorgang der Ultrafiltration führt zu einer gewünschten Entwässerung des Patientenbluts.

Bei der Hämofiltration wird dem Patientenblut durch Anlegen eines Transmembrandrucks im Dialysator Ultrafiltrat entzogen, ohne dass Dialysierflüssigkeit auf der dem Patientenblut gegenüber liegenden Seite der Membran des Dialysators vorbeigeführt wird. Zusätzlich kann dem Patientenblut eine sterile und pyrogenfreie Substituatslösung zugesetzt werden. Je nachdem, ob diese Substituatslösung stromaufwärts des Dialysators zugesetzt wird oder stromabwärts, spricht man von Prä- oder Postdilution. Der Stoffaustausch erfolgt bei der Hämofiltration konvektiv.

Die Hämodiafiltration kombiniert die Verfahren der Hämodialyse und der Hämofiltration. Es findet sowohl ein diffusiver Stoffaustausch zwischen Patientenblut und Dialysierflüssigkeit über die semipermeable Membran eines Dialysators statt, als auch eine Abfiltrierung von Plasmawasser durch einen Druckgradienten an der Membran des Dialysators.

Die Plasmapherese ist ein Verfahren, wonach das Patientenblut in das Blutplasma und seine korpuskularen Bestandteile (Zellen) aufgetrennt wird. Das abgetrennte Blutplasma wird gereinigt oder durch eine Substitutionslösung ersetzt und dem Patienten zurückgegeben.

Bei der Peritonealdialyse wird die Bauchhöhle eines Patienten über einen durch die Bauchdecke geführten Katheter mit einer Dialyseflüssigkeit befüllt, die ein Konzentrationsgefälle gegenüber den körpereigenen Flüssigkeiten aufweist. Über das als Membran wirkende Bauchfell (Peritoneum) treten die im Körper vorliegenden Giftstoffe in die Bauchhöhle über. Nach einigen Stunden wird die sich in der Bauchhöhle des Patienten befindliche, nunmehr verbrauchte Dialyseflüssigkeit ausgetauscht. Durch osmotische Vorgänge kann Wasser aus dem Blut des Patienten über das Bauchfell in die Dialyseflüssigkeit übertreten und den Patienten somit entwässern.

Die Verfahren zur Dialyse werden in der Regel mit Hilfe von automatischen Dialysegeräten, wie sie beispielsweise von der Anmelderin unter der Bezeichnung 5008 oder sleep.safe vertrieben werden, durchgeführt.

Diese Dialysegeräte sind komplexe medizinische Fluidmanagementgeräte mit umfangreichen Funktionen, für deren Bedienung und Überwachung, insbesondere bei Hämodialysegeräten, oftmals speziell geschultes Personal notwendig ist.

Bei der Hämodialyse hat sich deshalb die Behandlung in Dialysezentren, in Kliniken oder speziellen Dialysepraxen etabliert, bei der mehrere Dialysegeräte vorgehalten werden, mit denen mehrere Patienten gleichzeitig behandelt werden können.

Geschultes medizinisches Personal führt die Aufrüstung der Maschinen mit den notwendigen medizinischen Einmalartikeln, wie Schlauchsets, Dialysefilter und medizinischen Lösungen bzw. Konzentraten durch und stellt die Maschine entsprechend den ärztlichen Anweisungen für jeden Patienten individuell ein. Ebenso wird der Patient mit dem Dialysegerät verbunden und während der Dialyse ständig durch die Sicherheitseinrichtungen des Dialysegeräts und durch das medizinische Personal überwacht.

Zur Bedieneingabe und zur Informationsausgabe sind Dialysegeräte häufig mit Touchscreens ausgestattet. Die DE197 426 33A der Anmelderin zeigt hierfür ein Beispiel.

Dialysegeräte können mit umfangreichen Funktionen zur Überwachung der Behandlung und des Patienten ausgerüstet sein. Beispielsweise kann der Druck in dem arteriellen Blutschlauch, der Blut vom Patienten zum Dialysegerät führt, überwacht werden. Ein übermäßiges Absinken des arteriellen Blutdrucks kann beispielsweise auf eine Diskonnektierung der arteriellen Nadel hinweisen, oder auf eine Ansaugung der arteriellen Nadel an die Gefäßwand des Patienten. Beide Fälle begründen eine Alarmierung des medizinischen Personals, das weitere Schritte ausführen muss.

Aber auch bei Geräten für die automatische Peritonealdialyse, die oftmals, aber nicht zwingend, beim Patienten zuhause durchgeführt wird, können Situationen aufkommen, die einen Bedienereingriff einer geschulten Person, beispielsweise einer Dialyseschwester oder eines Dialysearztes notwendig machen.

Steht das Dialysegerät, wie häufig bei der Peritonealdialyse, beim Patienten zuhause, ist ein manueller Eingriff von medizinischem Personal nicht möglich.

In einer Dialysepraxis oder in einer Dialyseklinik stehen in der Regel mehrere Dialysegeräte in einem Raum und häufig auch in mehreren Räumen, die entfernt voneinander sind, oder auch über verschiedene Etagen verteilt sein können.

Ein Alarmzustand wird oftmals von einem Dialysegerät akustisch und optisch signalisiert, um das medizinische Personal auf einen entsprechenden Vorfall aufmerksam zu machen, beispielsweise im Stile einer Verkehrsampel.

Unabhängig davon werden Alarmzustände aber häufig auch akustisch durch Alarmtöne oder Alarmtonfolgen signalisiert, die eine vorgeschriebene Lautstärke haben müssen, damit das medizinische Personal sicher alarmiert wird.

Derartige akustische Alarme werden deshalb auch von anderen Patienten, die im gleichen Raum behandelt werden, gehört, was als störend empfunden werden kann. Die Dialysebehandlung dauert i.d.R. mehrere Stunden. Oftmals schlafen die Patienten während der Dialysebehandlung, so dass ein akustischer Alarm überdies zum Wecken von Patienten führt. Deswegen gibt die europäische Norm für elektrische medizinische Geräte DIN EN 60601-2-16 die Möglichkeit einer Fernalarmierung des medizinischen Personals, in dem ein Alarm zunächst nur an einem entfernten Gerät ausgegeben wird.

Kommt es zu einem Alarmzustand, muss sich das medizinische Personal bislang zum alarmierenden Gerät bewegen, um die Alarmursache zu evaluieren und zu beheben. Auch für regelmäßige Kontrollen der Maschine und des Befindens des Patienten muss sich das medizinische Personal bislang zum Patienten und dem Dialysegerät bewegen. Oftmals wird das medizinische Personal auch vom Patienten selbst gerufen, wenn er ein Anliegen hat.

In der täglichen Dialysepraxis bedeutet dies relativ weite Wege für das medizinische Personal und vermehrte Unruhe in den Behandlungsräumen, was unter therapeutischen und Komfortgesichtspunkten zu vermeiden ist.

Darüber hinaus kann es vorkommen, dass ein ärztlicher Eingriff in die Dialysegerätesteuerung notwendig ist. Ärzte sind aber nicht notwendigerweise ständig in räumlicher Nähe zu den Patienten zugegen. In einem solchen Fall vergeht unter Umständen wertvolle Zeit bis ein ärztlicher Eingriff stattfindet.

Der Erfindung liegt die Aufgabe zu Grunde, Verfahren und Vorrichtungen derart auszubilden, dass die Überwachung und die Steuerung von medizinischen Fluidmanagementgeräten von einem entfernten Ort möglich sind und deren Sicherheit und Bedienkomfort erhöht werden.

Die Lösung dieser Aufgaben erfolgt erfindungsgemäß durch ein Verfahren mit den Merkmalen des Anspruchs 1, durch eine System mit den Merkmalen des Anspruchs 7, und durch ein medizinisches Fluidmanagementgerät mit den Merkmalen des Anspruchs 13.

Vorteilhafte Ausführungsformen sind Gegenstand der Unteransprüche.

Die Erfindung beruht auf einem Verfahren zur entfernten Überwachung und Steuerung von medizinischen Fluidmanagementgeräten mit zumindest einer Ein- und Ausgabevorrichtung, vorzugsweise einem Touchscreendisplay, wobei zumindest Teile des Informationsgehaltes der zumindest einen Ein- und Ausgabevorrichtung des medizinischen Fluidmanagementgerätes zu zumindest einem entfernten Kontrollgeräts übertragen und ausgegeben werden, und wobei Bedienereingaben an zumindest einer Ein- und Ausgabevorrichtung des entfernten Kontrollgeräts, vorzugsweise einem Touchscreen, eingebbar sind, die das medizinische Fluidmanagementgerät steuern und wobei das medizinische Fluidmanagementgerät selbsttätig einen sicheren Zustand durch ein Abklemmen des extrakorporalen Blutkreislaufes vom vaskulären System des Patienten annimmt, wenn ein Verbindungsabbruch zwischen Fluidmanagementgerät und entferntem Kontrollgerät detektiert wird, wobei der Verbindungsabbruch dadurch detektiert wird, dass eine vom entfernten Kontrollgerät bei ordnungsgemäßem Empfang einer Übertragung vom medizinischen Fluidmanagementgerät an das medizinische Fluidmanagementgerät gesendete Bestätigungsmeldung vom medizinischen Fluidmanagementgerät nicht empfangen wird, oder wenn eine vom entfernten Kontrollgerät bei ordnungsgemäßem Empfang einer Übertragung vom medizinischen Fluidmanagementgerät an das medizinische Fluidmanagementgerät mit einer Zeitangabe gesendete Bestätigungsmeldung vom medizinischen Fluidmanagementgerät gegenüber einem Erwartungszeitpunkt verspätet empfangen wird.

Des Weiteren beruht die Erfindung auf einem System bestehend aus einem medizinischen Fluidmanagementgerät mit zumindest einer Ein- und Ausgabevorrichtung, vorzugsweise einem Touchscreendisplay und zumindest einem entfernten Kontrollgerät mit zumindest einer Ein- und Ausgabevorrichtung, vorzugsweise einem Touchscreendisplay, wobei das medizinische Fluidmanagementgerät und das entfernte Kontrollgerät dazu eingerichtet sind, Daten zu übertragen, wobei zumindest Teile des Informationsgehaltes der zumindest einen Ein- und Ausgabevorrichtung des medizinischen Fluidmanagementgeräts zu zumindest einem entfernten Kontrollgerät übertragen werden, und wobei das entfernte Kontrollgerät dazu eingerichtet ist, den vom medizinischen Fluidmanagementgerät übertragenen Informationsgehalt auf der zumindest einen Ein- und Ausgabevorrichtung anzuzeigen und Bedienereingaben an der zumindest einen Ein- und Ausgabevorrichtung an das medizinische Fluidmanagementgerät zu übertragen, und wobei das entfernte Kontrollgerät dazu eingerichtet ist, bei ordnungsgemäßem Empfang einer Übertragung vom medizinischen Fluidmanagementgerät eine Bestätigungsmeldung an das medizinische Fluidmanagementgerät zu senden, oder wobei das entfernte Kontrollgerät dazu eingerichtet ist, bei ordnungsgemäßem Empfang einer Übertragung vom medizinischen Fluidmanagementgerät eine Bestätigungsmeldung mit einer Zeitangabe an das medizinische Fluidmanagementgerät zu senden, wobei das medizinische Fluidmanagementgerät dazu eingerichtet ist, selbsttätig einen sicheren Zustand durch ein Abklemmen des extrakorporalen Blutkreislaufes vom vaskulären System des Patienten anzunehmen, wenn ein Verbindungsabbruch zwischen Fluidmanagementgerät und entferntem Kontrollgerät detektiert wird, wobei der Verbindungsabbruch dadurch detektiert wird, dass die Bestätigungsmeldung vom medizinischen Fluidmanagementgerät nicht, oder, wenn die Bestätigungsmeldung mit einer Zeitangabe gesendet wurde, gegenüber einem Erwartungszeitpunkt verspätet empfangen wird.

Des Weiteren beruht die Erfindung auf einem medizinischen Fluidmanagementgerät mit zumindest einer Ein- und Ausgabevorrichtung, vorzugsweise einem Touchscreendisplay, wobei das medizinische Fluidmanagementgerät dazu eingerichtet ist, Daten zu übertragen, wobei zumindest Teile des Informationsgehaltes der zumindest einen Ein- und Ausgabevorrichtung des medizinischen Fluidmanagementgeräts zu zumindest einem entfernten Kontrollgerät übertragen werden, und wobei das medizinische Fluidmanagementgerät dazu eingerichtet ist, selbsttätig einen sicheren Zustand durch Abklemmen des extrakorporalen Blutkreislaufes vom vaskulären System des Patienten anzunehmen, wenn ein Verbindungsabbruch zwischen Fluidmanagementgerät und einem entferntem Kontrollgerät detektiert wird, wobei der Verbindungsabbruch dadurch detektiert wird, dass eine vom entferntem Kontrollgerät bei ordnungsgemäßem Empfang einer Übertragung vom medizinischen Fluidmanagementgerät mit oder ohne Zeitangabe an das medizinische Fluidmanagementgerät gesendete Bestätigungsmeldung nicht, oder, wenn die Bestätigungsmeldung mit einer Zeitangabe gesendet wurde, gegenüber einem Erwartungszeitpunkt verspätet empfangen wird.

Aus dem Stand der Technik folgende Dokumente zur Überwachung und Steuerung von medizinischen Fluidmanagementgeräten von einem entfernten Ort bekannt:
WO 2009/122270 A1 richtet sich darauf, die Fernbedienbarkeit eines medizinischen Fluidmanagementgeräts zu unterbinden, wenn ein kritischer Betriebszustand beim medizinischen Fluidmanagementgerät vorliegt.

US 7 044 927 B2 zeigt ein System zur Fernsteuerung von extrakorporalen Blutbehandlungssystemen über LAN Verbindungen.

US 2011/066006 A1 zeigt ein System zur Übertragung von gemessenen Vitaldaten während einer Dialysebehandlung an ein entferntes Gerät und bedarfsweise ein Fernsteuern von Dialysegeräten von einem entfernten Kontrollgerät.

US 2010/137693 A1 und WO 2007/126360 A1 zeigen jeweils ein System mit einem medizinischen Fluidmanagementgerät und einem entfernten Kontrollgerät.

Im Folgenden soll stellvertretend für ein medizinisches Fluidmanagementgerät ein Dialysegerät, das als Hämodialysegerät oder als Gerät zur automatischen Peritonealdialyse ausgeführt sein kann, stehen. Dem Fachmann ist klar, dass die Erfindung ohne weiteres Zutun bei jedem medizinischen Fluidmanagementgerät zur Anwendung kommen kann.

Dialysegeräte sind häufig mit Touchscreenbedienerschnittstellen ausgerüstet, die als Ein- und Ausgabevorrichtungen dienen. Medizintechnische Geräte mit Touchscreens bieten dem Bediener eine komfortable, variable und durch die ebene und undurchbrochene Oberfläche eine hygienische Bedieneroberfläche.

Auf dem Touchscreen werden softwaregesteuert variable Bildinhalte angezeigt. So werden auf dem Touchscreen Einstellungen des Dialysegeräts, wie beispielsweise die Dialysierflüssigkeitsflussrate oder die vergangene Dialysedauer angezeigt. Ebenso können aktuelle Messwerte wie beispielsweise der arterielle und venöse Druck in den Zugangsschläuchen angezeigt werden. Durch Berührung des Touchscreens an Bedienflächen erfolgen Bedieneingaben für das Dialysegerät, um beispielsweise die Einstellung des Dialysegeräts zu verändern, oder auf einen Alarm entsprechend zu reagieren.

Erfindungsgemäß wird der Bildinhalt des Dialysegerätes an ein entferntes Kontrollgerät übermittelt. Diese Übermittlung kann beispielsweise kabelgebunden stattfinden, z.B. durch ein lokales Netzwerk (LAN), oder aber kabellos, beispielsweise über Funk (WLAN, Bluetooth) oder durch eine lichtgebundene Übertragung, beispielsweise per Infrarotschnittstelle.

Das entfernte Kontrollgerät ist bevorzugt ebenfalls mit einem Touchscreen ausgestattet, in jedem Fall aber mit einem Bildschirm und einer Eingabevorrichtung, beispielsweise einer Tastatur und/oder einer Computermaus.

Am entfernten Kontrollgerät wird der von dem Dialysegerät übermittelte Bildinhalt auf der Ausgabenvorrichtung ausgegeben. Es entsteht somit eine eins zu eins Abbildung des Bildinhalts des Touchscreens des sendenden Dialysegeräts.

Ist die Ein- und Ausgabevorrichtung des entfernten Kontrollgeräts als Touchscreen ausgeführt, können an diesem Bedieneingaben am Kontrollgerät analog zu Bedieneingaben an dem Dialysegerät erfolgen. Es ergibt sich dann kein Bedienunterschied zwischen dem Dialysegerät und dem entfernten Kontrollgerät.

Das entfernte Kontrollgerät kann aber auch ohne Touchscreen ausgeführt sein, beispielsweise als Notebook mit Touchpad oder angeschlossener Computermaus. Die Software des entfernten Kontrollgeräts ist in diesem Falle so programmiert, dass Bedieneingaben über die Tastatur und/oder über das Touchpad und/oder über die Computermaus realisiert werden. Beispielsweise können numerische Eingaben, die am Dialysegerät über eine angezeigte numerische Tastatur per Fingerdruck auf den Touchscreen eingegeben werden, auf einem Notebook per Tastatur komfortabel und schnell eingegeben werden.

Das entfernte Kontrollgerät kann auch ein SmartPhone oder ein Tablet-PC sein, insbesondere ein mobiles Computergerät, das Ärzte oder anderes medizinisches Personal mit sich tragen können.

Wesentlich für alle Ausführungen des entfernten Kontrollgeräts ist, dass Bedieneingaben an Bedienflächen des Bildschirminhaltes, der von dem sendenden Dialysegerät gesendet wurde, an der Ein- und Ausgabevorrichtung des entfernten Kontrollgeräts an jenes Dialysegerät übermittelt werden, welches den Bildschirminhalt gesendet hat, wo sie die gleiche Wirkung entfalten, als ob sie an diesem Dialysegerät eingegeben worden wären.

Auf diese Weise sind die Überwachung und die Steuerung von Dialysegeräten von einem entfernten Kontrollgerät in Echtzeit möglich und zwar in besonders vorteilhafter Weise von jedem Ort innerhalb der Reichweite des Netzwerkes, über welches die Dialysegeräte und das entfernte Kontrollgerät miteinander kommunizieren.

Findet diese Kommunikation nicht nur zwischen einem Dialysegerät und einem entfernten Kontrollgerät statt, sondern zwischen einem Dialysegerät und mehreren entfernten Kontrollgeräten, so kann eine Priorisierung der Bedieneingaben an den entfernten Kontrollgeräten vorgenommen werden, indem den entfernten Kontrollgeräten eine Wertigkeit zugeordnet wird, die sich an der Qualifikation des Bedieners orientiert.

So kann beispielsweise einem entfernten Kontrollgerät, welches sich in einem Schwesternzimmer befindet, eine niedrigere Wertigkeit zugeordnet werden, als einem entfernten Kontrollgerät, welches ein Dialysearzt bedient. Bedienereingaben von einem entfernten Kontrollgerät mit hoher Wertigkeit werden dann gegenüber Bedienereingaben von einem entfernten Kontrollgerät mit niedrigerer Wertigkeit bevorzugt ausgeführt.

Wenn beispielsweise der Dialysearzt über ein entferntes Kontrollgerät, welches ihm zugeordnet werden kann, eine Dialysebehandlung gestoppt hat, kann eine Dialyseschwester nicht ohne Weiteres diese Behandlung über eine Bedienereingabe an einem entfernten Kontrollgerät, welches ihr zugeordnet werden kann, wieder aufnehmen.

Die Zuordnung der entfernten Kontrollgeräte kann beispielsweise per Netzwerk-Adresse oder gerätespezifischer Hardwareadresse (MAC-Adresse) und hinterlegter Zuordnungstabelle erfolgen oder durch eine PKI-System (PKI, engl. public key infrastructure), welches digitale Zertifikate ausstellen, verteilen und prüfen kann. Alternativ oder zusätzlich kann sich das bedienende Personal am entfernten Kontrollgerät in geeigneter Weise authentifizieren, beispielsweise durch Eingabe einer persönliche Benutzername- und Passwortkombination (persönlicher alphanumerischer Code), oder durch das Einlesen des Fingerabdrucks über einen Fingerabdrucksensor oder durch das Einlesen eines Augenirisscans.

So kann sichergestellt werden, dass nur berechtigte Personen das Dialysegerät bedienen dürfen, und dass Bedieneingaben von Personen mit höherer fachlicher Kompetenz gegenüber Personen mit niedrigerer fachlicher Kompetenz bevorzugt ausgeführt werden.

Eine weitere Ausgestaltung der Erfindung sieht vor, dass neben der Übermittlung des Informationsgehaltes der zumindest einen Ein- und Ausgabevorrichtung eines Dialysegerätes auch Bild- und Tonsignale von Personen ausgetauscht werden können.

Hierzu sind zumindest die Dialysegeräte, bevorzugt auch die entfernten Kontrollgeräte mit einer Kamera und/oder einem Lautsprecher und/oder einem Mikrofon ausgestattet.

Die Kamera am Dialysegerät wird vorzugsweise auf den Dialysepatienten ausgerichtet. Im Bedarfsfall wird das aktuelle Bild des Patienten auf das entfernte Kontrollgerät übertragen, wo es angezeigt wird. Auf diese Weise kann das medizinische Personal den jeweiligen Patienten sehen.

Das entfernte Kontrollgerät kann ebenfalls mit einer Kamera und/oder einem Laut-sprecher und/oder einem Mikrofon ausgestattet sein, um das Bild des medizinischen Personals auf dem Bildschirm eines Dialysegeräts anzuzeigen und/oder eine verbale Kommunikation zu ermöglichen.

Vorzugsweise erfolgt die Anzeige der Kamerabilder auf Teilflächen der Ausgabevorrichtungen.

Die Initiierung visueller oder verbaler bzw. akustischer Kommunikation kann hierbei sowohl vom Dialysegerät als auch vom entfernten Kontrollgerät erfolgen, indem ein entsprechender Bedieneingriff am Gerät selbst oder einem mit dem Gerät verbundenen externen Gerät, wie beispielsweise einer Fernsteuerung oder einem Patienteninterface erfolgt.

Auf diese Weise können Patient und Arzt bzw. medizinisches Personal miteinander sprechen und sich gegenseitig auf dem jeweiligen Bildschirm sehen. So können Dialysepatienten ihre Bedürfnisse dem medizinischen Personal mitteilen, ohne darauf warten zu müssen, dass das medizinische Personal sich in Rufnähe zu ihnen befindet. Umgekehrt kann das medizinische Personal mit einem Patienten sprechen, um ihn beispielsweise nach seinem Befinden zu befragen, ohne sich zu ihm bewegen zu müssen.

Dies bedeutet eine große Arbeitserleichterung und spart unnötige Wege in einer Behandlungsstation, was wesentlich zur Ruhe und dem Komfort in der Behandlungsstation beiträgt.

Die Kommunikation durch Kamera, Mikrofon und Lautsprecher kann nicht nur zwischen medizinischem Personal und Patienten oder auf zwei Teilnehmern beschränkt erfolgen. Denkbar ist beispielsweise auch die Kommunikation zwischen Patienten untereinander. Im Arbeitsablauf einer Dialysestation kann es vorkommen, dass befreundete Patienten, die zur gleichen Zeit behandelt werden, an voneinander weit entfernten Dialysegeräten behandelt werden.

Durch die erfindungsgemäße Ausgestaltung der Dialysegeräte mit Kamera, Lautsprecher und Mikrofon können die Patienten auch untereinander kommunizieren. Vorteilhaft ist für eine solche Kommunikation, wenn die Dialysegeräte zusätzlich mit einem mobilen, tragbaren Patienteninterface ausgerüstet sind, welches für den Dialysepatienten vorgehalten wird. Ein solches Patienteninterface kann beispielsweise ein Notebook oder ein Tablet-PC sein, welche bevorzugt drahtlos mit der Dialysemaschine oder einem entfernten Server datentechnisch verbunden sind und dem Behandlungsplatz eindeutig zugeordnet werden können. Die Ausgabevorrichtung des mobilen, tragbaren Patienteninterface ist bevorzugt ein Touchscreen.

Das mobile, tragbare Patienteninterface kann ebenfalls mit einer Kamera und/oder einem Lautsprecher und/oder einem Mikrofon ausgestattet sein. Das Dialysegerät ist in der Regel so aufgestellt, dass die Ausgabevorrichtung vom medizinischen Personal bequem ablesbar ist. Das bedeutet oftmals, dass der behandelte Patient nur eine eingeschränkte oder gar keine Sicht auf die Ausgabevorrichtung des Dialysegeräts hat. Häufig ist die Information auf der Ausgabevorrichtung des Dialysegeräts für den Patienten zudem uninteressant.

Wird das Patienteninterface als tragbares Gerät ausgeführt, kann der Patient das Patienteninterface während der Dialyse bequem so halten, dass die Ausgabevorrichtung gut ablesbar ist. Der Bildinhalt der Ausgabevorrichtung des Patienteninterfaces kann unabhängig vom Bildinhalt der Ausgabevorrichtung des Dialysegeräts sein. So können beispielsweise Multimediainhalte wie Filme oder Bilder angezeigt werden, denkbar ist aber auch ein Webbrowser, der Zugang zum Internet ermöglicht. Somit wird der Patient während der Dialysebehandlung individuell unterhalten und informiert. Um andere Patienten in einer Dialysestation nicht zu stören, kann das Patienteninterface mit einem Kopfhörer ausgestattet sein.

Für die Kommunikation zwischen Patienten kann es vorgesehen sein, dass eine spezielle Software vorgehalten wird, die ähnlich einer Vermittlung alle an der Patient-zu-Patient Kommunikation teilnehmenden Patienten auflistet und auswählbar macht. Denkbar ist, dass einem Patient durch entsprechende Bedienereingaben auf seinem Patienteninterface ein Auswahlfenster angezeigt wird, auf dem er per Bedienereingabe eine Verbindung zwecks Kommunikation zu anderen Patienten herstellen kann.

Eine weitere erfindungsgemäße Ausführungsform sieht vor, dass das entfernte Kontrollgerät ein Smartphone ist, welches das medizinische Personal, insbesondere ein Arzt, mit sich trägt. Smartphones zeichnen sich gegenüber normalen mobilen Telephonen durch ihre erweiterte Computerfunktionalität aus. Diese können sich beispielsweise in der Vorhaltung eines hochauflösenden Displays, bevorzugt mit Touchscreenfunktionalität, äußern. Die Betriebssysteme von Smartphones sind in der Regel derart ausgelegt, dass vielfältige Programme, sogenannte Apps, ausgeführt werden können. Unter den Smartphone-Geräten werden im Erfindungssinne alle mobilen Geräte verstanden, die Computerfunktionalität haben, aber keine Computer im Sinne von Laptops oder Notebooks sind, beispielsweise auch sogenannte Tablet-PCs.

Smartphones können in der Regel sowohl Daten über das Mobiltelefonnetz als auch über lokale Funknetze (WLAN, Bluetooth) austauschen. Ein Arzt, der ein solches Smartphone mit sich führt, kann von überall aus in schon beschriebener Weise Zugriff auf ein bestimmtes Dialysegerät haben. Smartphones sind in der Regel auch mit einer Kamera ausgestattet, so dass auch bei dieser Ausführungsform eine direkte Kommunikation mit Bildübertragung in schon beschriebener Weise möglich ist.

Besonders vorteilhaft ist diese Ausführung bei Patienten, die eine sogenannte Heimdialyse durchführen, beispielsweise bei der Peritonealdialyse oder bei Hämodialysegeräten für den Heimbetrieb. In solchen Fällen führt der Patient die Dialyse zuhause durch, und in der Regel ist dabei kein medizinisches Fachpersonal anwesend. Dabei kann es zu Situationen kommen, in denen ein fachkundiger Eingriff in die Gerätesteuerung notwendig ist. Erfindungsgemäß kann ein solcher Eingriff nun von dem medizinischen Personal durch das entfernte Kontrollgerät, welches ein Smartphone sein kann, ausgeführt werden. Darüber hinaus kann auch die Kommunikation in schon beschriebener Weise über das Mobilfunknetz oder über das Internet erfolgen.

Wesentlich für die Sicherheit der entfernten Kontrolle eines Dialysegeräts ist, dass es zu keiner Fehlübertragung von Bildinhalten vom Dialysegerät zum entfernten Kontrollgerät und/oder zur fehlerhaften Übertragung von Bedienereingaben vom entfernten Kontrollgerät zum Dialysegerät kommt.

Hierzu werden umfangreiche Sicherheitsvorkehrungen getroffen. Es ist vorgesehen, dass der Bildinhalt eines Dialysegeräts periodisch zum entfernten Kontrollgerät übertragen wird. Es erfolgt also ein periodischer Screenupdate. Zusätzlich können Screenupdates erfolgen, wenn sich der Bildinhalt ändert, beispielsweise wenn sich ein Messwert, der auf dem Bildschirm des Dialysegeräts angezeigt wird, ändert.

Um sicherzustellen, dass das übertragene Bild auf dem entfernten Kontrollgerät den gleichen Inhalt darstellt wie auf dem Dialysegerät, kann zu jedem Screenupdate auch ein CRC (Cyclic Redundancy Check) über den Bildschirminhalt berechnet und mit übertragen werden. Die zyklische Redundanzprüfung (CRC) ist ein Verfahren zur Bestimmung eines Prüfwerts für Daten, um Fehler bei der Übertragung oder Speicherung erkennen zu können. Im entfernten Kontrollgerät kann ebenfalls ein CRC über den empfangenen Bildschirminhalt berechnet und mit den empfangenen CRC-Daten verglichen werden. Bei fehlerfreier Übertragung unterscheiden sich die beiden CRC Daten nicht. Wird ein Unterschied zwischen den von dem Dialysegerät übertragenen CRC-Daten und den von dem entfernten Kontrollgerät berechneten CRC-Daten festgestellt, liegt offensichtlich ein Übertragungsfehler vor. Das entfernte Kontrollgerät kann dann eine neue Übertragung beim entsprechenden Dialysegerät anfordern. Bleibt die Übertragung weiterhin fehlerhaft, kann das entfernte Kontrollgerät ein Fehlermeldungssignal (optisch, akustisch oder haptisch) ausgeben, das das medizinische Personal zum Überprüfen der betroffenen Dialysemaschine veranlassen soll.

Zusätzlich zur Übertragung des Bildinhalts zusammen mit den Daten aus einem CRC kann eine Zeitangabe mit übertragen werden (timestamp), die darüber Auskunft gibt, welche Tageszeit der übertragene Bildinhalt zugeordnet ist. Das entfernte Kontrollgerät vergleicht bei Empfang eines neuen Bildinhalts diesen mit übertragenem Timestamp mit dem letzten empfangenen Timestamp und der aktuellen Zeit. Durch die vorgesehene periodische Übertragung in äquidistanten Zeitintervallen bei normalem Betrieb kann das entfernte Kontrollgerät bestimmen, ob die aktuell empfangene Übertragung die im Vergleich zur letzten empfangenen Übertragung Erwartete ist, oder ob die aktuelle Übertragung verfrüht kommt, was auf eine Änderung des Bildschirminhalts, beispielsweise einen veränderten Messwert, hinweist, die zeitlich vor dem normalen Screenupdate erfolgt. Solche Übertragungen können besonders behandelt werden, beispielsweise kann die Veränderung zum letzten übertragenen Bildschirminhalt gesondert kenntlich gemacht werden, beispielsweise durch graphische Hervorhebung, wie einen Hinweispfeil, farbliche Hervorhebung, Blinken oder Anzeige in einem gesonderten Bereich der Ausgabevorrichtung des entfernten Kontrollgeräts.

Auf die gleiche Art kann das entfernte Kontrollgerät feststellen, dass eine erwartete Übertragung ausbleibt. In diesem Fall kann das entfernte Kontrollgerät über das Netzwerk ein Screenupdate bei dem fraglichen Dialysegerät anfordern. Bleibt dieses Screenupdate aus, kann eine Fehlermeldung erfolgen, die das medizinische Personal veranlasst, das Dialysegerät zu überprüfen und gegebenenfalls weitere Schritte zu veranlassen.

Es ist genauso wesentlich, dass sichergestellt wird, dass das entfernte Kontrollgerät ordnungsgemäß arbeitet und den von einem Dialysegerät übertragenem Bildschirminhalt auch ordnungsgemäß anzeigt. Hierzu kann das entfernte Kontrollgerät bei ordnungsgemäßem Empfang einer Übertragung eine Bestätigungsmeldung an das betreffende Dialysegerät senden, welche ebenfalls mit einer Zeitangabe versehen sein kann. Bleibt diese Bestätigungsmeldung aus, oder kommt verspätet, deutet das auf Probleme bei der Übertragung hin, und das Dialysegerät kann entsprechende Fehlermeldungen und/oder Alarmsignale melden. Durch die beidseitige Überprüfung der Übertragung wird die Sicherheit noch mehr erhöht.

Es ist darüber hinaus wesentlich, dass sichergestellt wird, dass Bedienereingaben am entfernten Kontrollgerät fehlerfrei an ein Dialysegerät übertragen werden. Dies kann dadurch erreicht werden, indem das Dialysegerät vor Ausführung einer mit der Bedienereingabe verbundenen Aktion die empfangene Bedienereingabe von einem entfernten Kontrollgerät an das entfernte Kontrollgerät zurücksendet, wo verglichen wird, ob gesendete und wieder empfangene Bedienereingabe übereinstimmen. Abhängig von diesem Vergleich wird vom entfernten Kontrollgerät ein Signal an das Dialysegerät gesendet, das entweder die mit der Bedienereingabe verbundene Aktion freigibt, oder sie verbietet. Das Dialysegerät kann nach Ausführen der mit der Bedienereingabe verbundenen Aktion ein Bestätigungssignal an das entfernte Kontrollgerät senden, aus dem hervorgeht, dass die Aktion ausgeführt worden ist. Kommt es bei diesem Ablauf an irgendeiner Stelle zu Abweichungen vom Erwarteten, wird an dem entfernten Kontrollgerät und/oder an dem Dialysegerät ein Alarmsignal (akustisch, optisch oder haptisch) gegeben. Das medizinische Personal kann darauf hin die Geräte überprüfen, die Fehlerursache beseitigen und den Alarm quittieren.

Bei allen möglichen Ausführungsbeispielen werden für die Übertragung von Daten solche Datenübertragungsprotokolle bevorzugt, die sicher bezüglich der Vertraulichkeit, der Authentizität und Integrität sind, wie beispielsweise die Datenübertragungsprotokolle Internet Protocol Security (IPSec) oder Virtual Private Network (VPN, OpenVPN).

Weiterhin kann zur Authentifizierung des zu bedienenden Dialysegeräts und des entfernten Kontrollgeräts ein Datenübertragungsprotokoll mit sicherer Identifikation der sich austauschenden Geräte, wie beispielsweise Bluetooth oder Infrarot (IrDA=Infrared Data Association) verwendet werden.

Es kann vorgesehen sein, dass bei der initialen Kontaktaufnahme zwischen dem zu bedienenden Dialysegerät und dem entfernten Kontrollgerät eindeutige Identifikationsmerkmale der kommunizierenden Geräte wie MAC-Adressen oder Geräteidentifikationsnummern ausgetauscht werden. Diese Identifikationsmerkmale können mit unterschiedlichen Legitimationen verknüpft sein. Je nach Legitimation kann die Datenübertragung erlaubt, blockiert oder eingeschränkt werden. Diese Vorgehensweise stellt sicher, dass Informationen vom Dialysegerät nur an Personen übertragen werden, die hierfür berechtigt sind.

Durch die sichere Identifikation des zu bedienenden Dialysegeräts und des entfernten Kontrollgeräts kann in einfacher Weise ein einziges entferntes Kontrollgerät zur Bedienung mehrerer unterschiedlicher Dialysegeräte verwendet werden. Hierbei ist sichergestellt, dass der Bediener am entfernten Kontrollgerät das zu bedienende Dialysegerät eindeutig identifiziert, beispielsweise durch die Anzeige einer Geräteidentifikationsnummer.

Um Energie beim Dialysegerät einzusparen, kann dessen Touchscreendisplay überwiegend dunkel geschaltet, oder mit reduzierter Helligkeit betrieben werden. Erst im Bedienfall oder bei Alarmmeldungen kann vorgesehen sein, das Display im Normalmodus zu betreiben. Die Bedienbarkeit des Dialysegeräts und die Bereitstellung von Informationen können hierbei durch die erfindungsgemäße Datenübertragung an ein entferntes Kontrollgerät erhalten bleiben. In einer Dialysestation mit einer Vielzahl von Dialysegeräten kann auf diese Weise beträchtlich Energie eingespart werden, was sich positiv auf die Betriebskosten auswirkt.

Es kann bei allen Ausführungsbeispielen vorgesehen sein, dass im Falle eines Verbindungsabbruchs zwischen Dialysegerät und entferntem Kontrollgerät oder im Fall von fehlerhafter Verbindung das Dialysegerät selbständig in einen sicheren Betriebszustand übergeht, um den Patienten nicht zu gefährden und die Sicherheit zu erhöhen. Ein solcher sicherer Zustand ist beispielsweise ein Abklemmen des extrakorporalen Blutkreislaufes vom vaskulären System des Patienten durch entsprechende Klemmen an dem arteriellen und venösen Zugang des Patienten und ein voller Stopp der Blutpumpe.

Eine weitere Ausgestaltung der Erfindung sieht vor, dass Alarmzustände, die ein Dialysegerät detektiert, zunächst dem entfernten Kontrollgerät übermittelt werden, ohne bei dem Dialysegerät zu einer unmittelbaren Ausgabe eines optischen, akustischen oder haptischen Alarmsignal zu führen. Üblicherweise sind Dialysegeräte mit einer Vielzahl von Überwachungsvorrichtungen ausgerüstet, die die Dialysebehandlung überwachen und im Fehlerfall Alarmsignale aussenden. Diese Alarmsignale können optisch, akustisch und haptisch sein. Wesentlich für die Alarmsignale ist, dass sie vom medizinischen Personal bemerkt werden. In Dialysezentren wird aber beim Aussenden eines Alarmsignals nicht nur das medizinische Personal alarmiert, sondern auch die anwesenden Patienten durch das Alarmsignal gestört. Um diese Störung zu vermeiden, sieht die Erfindung vor, einen auftretenden Alarmzustand zunächst am entfernten Kontrollgerät zu signalisieren.

Dadurch wird zunächst das medizinische Personal alarmiert, welches sich zum betreffenden Dialysegerät bewegen muss, um die Ursache des Alarms zu beheben. Das medizinische Personal kann in weiterer Folge den Alarm an dem Dialysegerät quittieren. Es kann vorgesehen sein, dass das Dialysegerät auf eine ausbleibende Quittierung des Alarms mit dem verzögerten Signalisieren des Alarms an dem Dialysegerät selbst reagiert.

Weitere Einzelheiten und Vorteile der Erfindung werden anhand von in den Zeichnungen dargestellten Ausführungsbeispielen näher beschrieben. Es zeigen:
Figur 1 eine Dialysestation mit erfindungsgemäßer Vorrichtung zur entfernten Überwachung und Steuerung von Dialysegeräten;
Figur 2 eine erste Ausführungsform des Bildschirminhalts eines erfindungsgemäßen entfernten Kontrollgeräts zur entfernten Überwachung und Steuerung von Dialysegeräten;
Figur 3 eine weitere Ausführungsform des Bildschirminhalts eines erfindungsgemäßen entfernten Kontrollgeräts zur entfernten Überwachung und Steuerung von Dialysegeräten und
Figur 4 ein als Smartphone ausgeführtes erfindungsgemäßes entferntes Kontrollgerät zur entfernten Überwachung und Steuerung von Dialysegeräten.

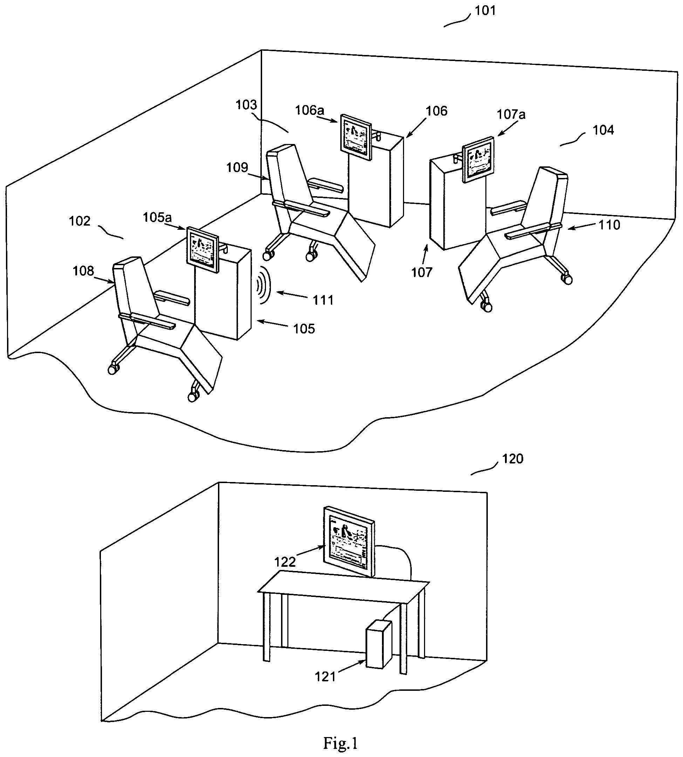

In Figur 1 ist eine typische Dialysestation mit Behandlungsraum 101 und einem Überwachungsraum 120 dargestellt, die voneinander entfernt sein können. Im Behandlungsraum 101 sind drei Behandlungsstationen 102, 103, 104, die jeweils aus Dialysegerät 105, 106, 107 und Patientenlagerungsvorrichtung 108, 109, 110, hier als Liege ausgeführt, bestehen. Die Dialysegeräte 105, 106, 107 sind jeweils mit einer Ausgabevorrichtung 105a, 106a, 107a ausgestattet, die hier exemplarisch als Touchscreen ausgeführt sind.

Im Überwachungsraum 120 wird ein entferntes Kontrollgerät, hier als stationärer Computer 121 mit Touchscreen 122 ausgeführt, vorgehalten. Im Überwachungsraum hat das medizinische Personal die Möglichkeit, die Dialysebehandlung eines Patienten im entfernten Behandlungsraum 101 zu überwachen und/oder auf die Gerätesteuerung Einfluss zu nehmen. In Figur 1 ist durch die konzentrischen Kreisausschnitte 111 angedeutet, wie das Dialysegerät per Funk, beispielsweise über eine abgesicherte WLAN Verbindung, Daten an das entfernte Kontrollgerät 121 sendet. Das entfernte Kontrollgerät kann daraufhin den Bildschirminhalt, oder Teile davon auf seiner Ausgabevorrichtung 122 anzeigen. Darüber hinaus werden auf dem Touchscreen 122 auch die Bedienflächen des sendenden Dialysegeräts 105 angezeigt. Das medizinische Personal kann durch Drücken auf diese Bedienflächen das sendende Dialysegerät in gleicher Weise bedienen, als wenn die Bedieneingabe auf dem Touchscreen 105a erfolgt wäre. Hierzu kommuniziert das entfernte Kontrollgerät 121 in schon beschriebener Weise mit dem Dialysegerät 105a, welches die Bedienereingaben am entfernten Kontrollgerät umsetzt.

Die Dialysegeräte 105, 106, 107 können mit einer Kamera und einem Mikrofon in schon beschriebener Weise ausgerüstet sein (in Figur 1 nicht eingezeichnet). Vorzugsweise ist die Kamera beweglich so an dem Dialysegerät oder an einem Patienteninterface, das mit dem Dialysegerät interagiert, angebracht, dass sie sich auf den Patienten ausrichten lässt. Alternativ können aber auch eine oder mehrere bewegliche Kameras beispielsweise an der Decke des Behandlungsraums 101 angebracht werden, die sich per Steuerung auf einen Patienten richten lassen. Wesentlich ist, dass Kameras vorgehalten werden können, mit denen das Bild eines bestimmten Patienten an das entfernte Kontrollgerät gesendet werden kann.

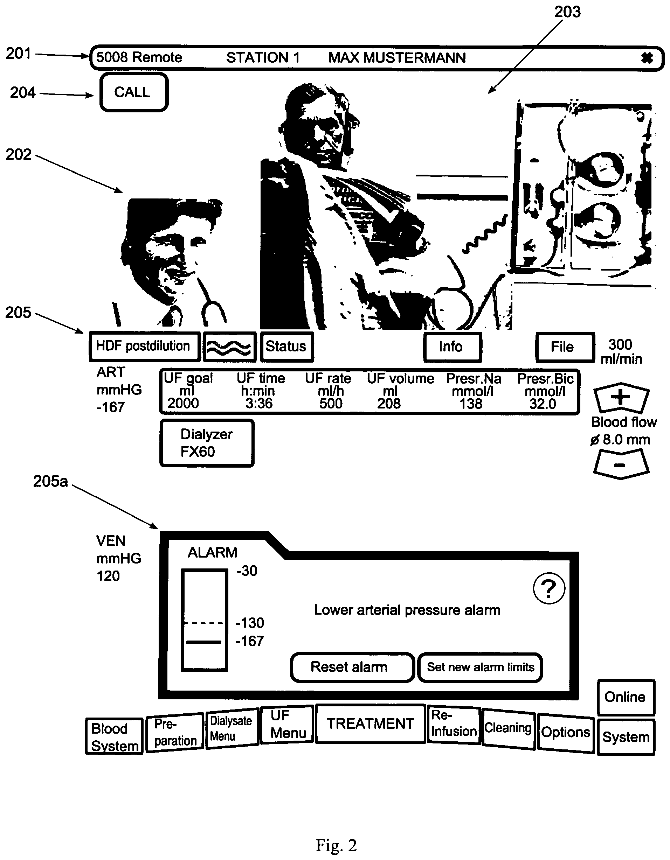

In Figur 2 ist ein Beispiel für den angezeigten Bildschirminhalt eines Dialysegeräts auf der Ausgabevorrichtung eines entfernten Kontrollgeräts dargestellt.

Die Bildschirmanzeige besteht aus den Informationen in einem Anzeigebereich 201, welches Dialysegerät (in Figur 2 das Dialysegerät 5008) in welchem Behandlungsraum (Station 1) mit welchem Patienten (Max Mustermann) gerade durch das entfernte Kontrollgerät überwacht und gesteuert wird. Unter dem Anzeigebereich 201 sind die aktuellen Bilder 202 und 203 des medizinischen Personals am entfernten Kontrollgerät und des Patienten am betreffenden Dialysegerät zu sehen. Durch Fingerdruck auf die Bedienereingabe 204 (CALL) kann eine audiovisuelle Verbindung zwischen medizinischem Personal und Patienten hergestellt werden. Dies geschieht in schon beschriebener Weise über Kameras, Lautsprecher und Mikrofone am Dialysegerät und am entfernten Kontrollgerät.

Unterhalb der Bilder des medizinischen Personals und des Patienten wird die übertragene eins zu eins Abbildung 205 des Bildschirminhalts des Dialysegeräts angezeigt. Diese Anzeige ist zur gleichen Zeit auch auf der Ausgabevorrichtung des betreffenden Dialysegeräts zu sehen. Es handelt sich hierbei um die Anzeige eines Alarmzustandes, nämlich dem der Unterschreitung des arteriellen Druckes. Dabei erfolgt die Alarmmeldung in einem speziellen Bereich 205a des Anzeigebereichs 205. Das medizinische Personal erhält in diesem Bereich die Möglichkeit entweder den Alarm zurückzusetzen (in Figur 2 durch die Bedienfläche "Reset alarm" dargestellt), oder eine neue Untergrenze für den arteriellen Druck einzugeben (in Figur 2 durch die Bedienfläche "Set new alarm limits" dargestellt). Auf die gleiche Weise kann jede Bedienereingabe für ein bestimmtes Dialysegerät, auch solche, denen keine Alarmmeldung vorangegangen ist, am entfernten Kontrollgerät erfolgen.

Es kann überdies am entfernten Kontrollgerät auch ein zusätzliches akustisches, optisches oder haptisches Alarmsignal ausgegeben werden, um das medizinische Personal aufmerksam zu machen. Dieses Alarmsignal kann am betreffenden Dialysegerät unterdrückt werden, um den betreffenden Patienten nicht zu beunruhigen und Patienten, die im gleichen Raum behandelt werden, nicht zu stören.

Es kann vorkommen, dass bestimmte Situationen auftreten, in denen sich das medizinische Personal zu einem bestimmten Dialysegerät bewegen muss. Beispiele hierfür können sein, wenn der Patient ein Anliegen hat oder, wenn manuelle Eingriffe am Gerät, beispielsweise das Abkoppeln des Patienten von dem Dialysegerät, notwendig sind.

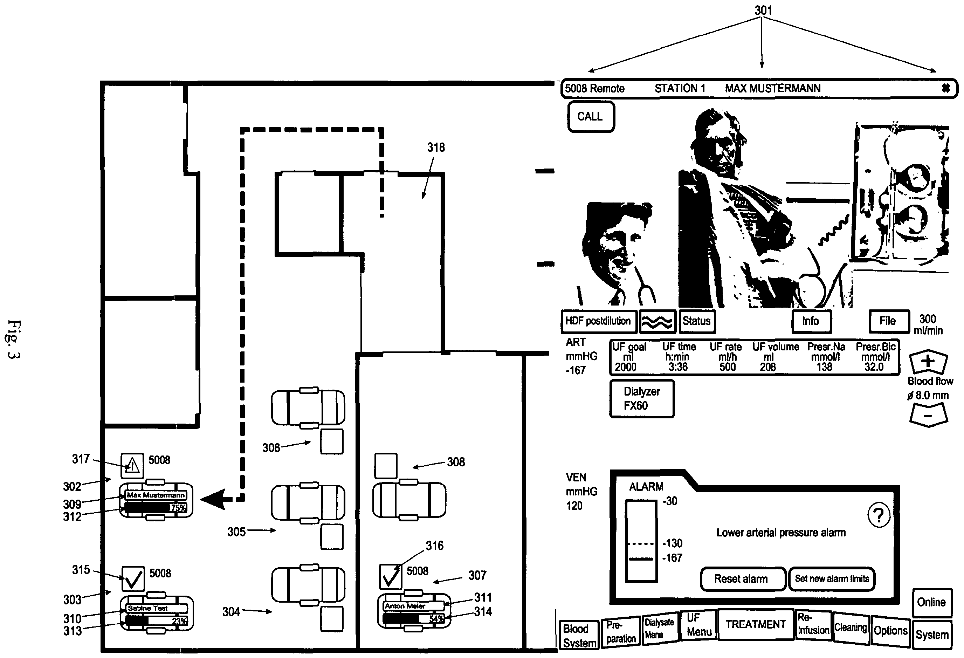

In Figur 3 ist dargestellt, wie erfindungsgemäß die Fernüberwachung und entfernte Steuerung mehrerer Dialysegeräte innerhalb einer Dialysestation erleichtert wird. Der rechte Teil 301 der Figur 3 entspricht der Anzeige wie in Figur 2 dargestellt. Zusätzlich wird ein Lageplan der Dialysestation im linken Teil der Figur 3 angezeigt. Dem medizinischen Personal wird hierdurch ein Überblick gegeben, wie die Station aktuell durch welche Patienten belegt ist, an welchem Dialysegerät diese Patienten gerade behandelt werden, wo sich diese Dialysegeräte in der Dialysestation befinden und wie der Status der Behandlung aktuell ist. Die gesamte Figur 3 ist ein Beispiel für eine Bildschirmanzeige auf einem entfernten Kontrollgerät, beispielsweise an einem großen Monitor mit Touchscreenfunktionalität in einem Überwachungsraum einer Dialysestation.

Es ist in Figur 3 durch eine Aufsicht gezeigt, wo sich die Dialysegeräte 302 bis 308 befinden, und ob diese Geräte aktuell gerade benutzt werden. Hierzu sind die Dialysegeräte mit Patientenlagervorrichtung in einen Lageplan der Dialysestation eingezeichnet. Wird ein Patient aktuell gerade an einem bestimmte Dialysegerät behandelt, kann sein Name in einem Anzeigebereich (309, 310, 311) innerhalb der symbolisierten Patientenlagervorrichtung angezeigt werden. Ebenso kann der Fortschritt der Dialysebehandlung angezeigt werden. In Figur 3 erfolgt das durch die Balkenanzeigen 312, 313, 314 mit jeweiliger Prozentangabe der verstrichenen Dialysezeit an der Gesamtdialysedauer, denkbar sind aber auch andere Darstellungsarten.

In Figur 3 werden im jeweiligen symbolisierten Dialysegerät Häkchen 315, 316 angezeigt, wenn die Behandlung innerhalb normaler Parameter abläuft, oder Ausrufezeichen 317, wenn ein Problem auftritt. Denkbar sind auch beliebige andere Zeichen und Anzeigestellen. Wichtig ist nur, dass dem Betrachter eindeutig signalisiert wird, ob ein bestimmtes Dialysegerät aktuell ein Problem aufwirft, oder ob die Behandlung an einem bestimmten Dialysegerät aktuell ohne Problem abläuft.

Durch den in Figur 3 unterbrochen eingezeichneten Pfeil, der seinen Ursprung in dem Überwachungsraum 318 hat und der auf das Dialysegerät 302 zeigt, wird dem medizinischen Personal eindeutig gezeigt, an welches Dialysegerät es sich im Bedarfsfall bewegen muss.

Überdies wird durch den Pfeil eine Art Wegweiser realisiert, der es dem Personal in großen Dialysestationen mit vielen Behandlungsorten erleichtert, ein bestimmtes Dialysegerät zu finden. Für die Darstellung des Wegweisers sind beliebige graphische Anzeigen denkbar.

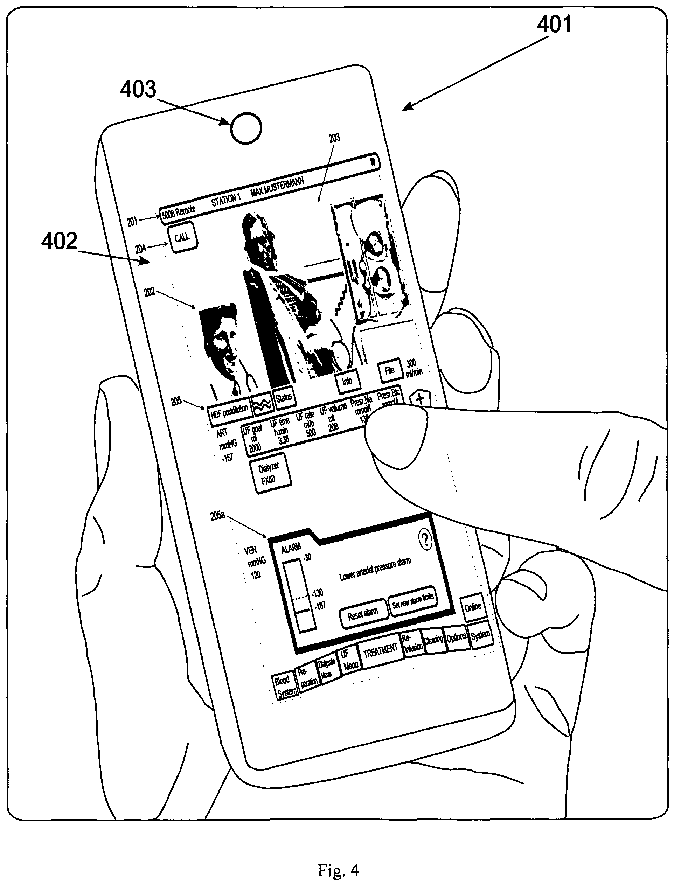

Vorstellbar ist, dass das Personal zusätzlich über ein tragbares Gerät wie in Figur 4 dargestellt verfügt. Auf diesem könnte der Weg zu einer bestimmten Dialysestationen abhängig von der Position des medizinischen Personals ähnlich einem Navigationsgerät dargestellt werden. Das Gerät gibt dem Betrachter also Hinweise in Form von beispielsweise Pfeilen und/oder akustischen Richtungsangaben, wohin der Betrachter sich wenden muss, um zu einem bestimmten Dialysegerät zu gelangen. Somit würde das medizinische Personal auf dem kürzesten Weg zu einem Patienten geleitet.

Vorstellbar ist auch, dass ein solcher Wegweiser auf tragbaren Geräten von medizinischem Personal angezeigt wird, das die Dialysebehandlung nicht betreut, sondern möglicherweise wegen einer anderen Indikation während einer Dialysebehandlung medizinische Handlungen an einem bestimmten Dialysepatienten vornehmen muss. So könnte beispielsweise bei schwangeren Dialysepatientinnen im Bedarfsfall ein Frauenarzt herbeigerufen werden, der sich anhand des angezeigten Wegweisers auf einem tragbaren Gerät, wie beispielsweise einem Smartphone, schnell orientieren kann und den Patienten sicher und zeitnah erreicht. Die Positionsbestimmung des entfernten Kontrollgeräts kann hierbei durch von Navigationssystemen bekannte Methoden erfolgen, beispielsweise durch die Nutzung einer GPS basierenden Positionsbestimmung. Denkbar ist aber auch die Auswertung der Signalstärken von Mobilfunk- oder WLAN-Sendern.

In der Figur 4 ist ein Ausführungsbeispiel für ein tragbares entferntes Kontrollgerät 401 gezeigt, das als Smartphone ausgeführt ist. Im Wesentlichen unterscheidet sich die Anzeige 402 auf einem tragbaren entfernten Kontrollgerät nicht von den Anzeigen auf den Ausgabevorrichtungen eines stationären entfernten Kontrollgeräts. Die Ausführung als Smartphone macht es aber möglich, medizinisches Personal an der Überwachung und der Steuerung von Dialysegeräten zu beteiligen, das sich nicht in der fußläufig erreichbaren Nähe der Dialysegeräte befindet. Vorstellbar ist, dass beispielsweise Heimdialysepatienten derart durch medizinisches Personal über ein entferntes Kontrollgerät überwacht werden. Hierbei spielt es keine Rolle, welcher Art der Dialyse sich der Patient zuhause unterzieht.

Vorstellbar ist auch, dass beispielsweise ein Dialysearzt mit einem solchem tragbaren Kontrollgerät ausgestattet wird und sich per Mobilfunknetz an der Überwachung und Steuerung von Dialysegeräten beteiligen kann. Oftmals sind solche mobilen Kontrollgeräte mit einer Kamera ausgerüstet, wie in Figur 4 durch das Kameraobjektiv 403 gezeigt. Die Ausstattung mit einem Lautsprecher und einem Mikrofon ist ohnehin bei Smartphones Standard, jedoch auch Tablet-PCs, als weiteres Ausführungsbeispiel für ein tragbares entferntes Kontrollgerät, sind in der Regel mit Kamera, Mikrofon und Lautsprecher ausgerüstet. Smartphones und Tablet-PCs sind in der Regel mit Touchscreens ausgestattet. Vorstellbar ist aber auch, dass das entfernte, mobile Kontrollgerät ein Laptop, Notebook oder Netbook mit Tastatur ist, das mit einem Touchpad und/oder einer Computermaus ausgestattet sein können.

Durch die Mobilität des entfernten Kontrollgeräts in der in Figur 4 gezeigten Ausführungsform kann das medizinische Personal jederzeit und überall kontaktiert werden. Vorstellbar ist, dass das normale betreuende medizinische Personal bevorzugt in fußläufiger Nähe zu den Patienten der Überwachung der Dialysegeräte nachgeht, Dialyseärzte aber nicht notwendigerweise in fußläufiger Nähe zu den Patienten zugegen sind. Im Bedarfsfalle kann der Rat eines Spezialisten, bevorzugt eines Dialysearztes bzw. Nephrologen auf diese Weise eingeholt werden. Der Spezialist kann sich hierbei überall auf der Welt im Bereich eines unterstützen Mobilfunknetzes befinden, oder eine Internetverbindung nutzen. Dem Spezialisten stehen durch die Übertragung des Bildschirminhaltes und der Bedienflächen umfangreiche Möglichkeiten zur Steuerung des Dialysegeräts zur Verfügung. Es ist vorstellbar, dass die Kontaktierung eines Spezialisten durch ein mobiles entferntes Kontrollgerät von dem Dialysegerät selbstständig initiiert wird. Dies kann beispielsweise bei bestimmten Alarmsituationen oder bei bestimmten Patienten erfolgen, denen, beispielsweise aufgrund einer besonderen medizinischen Indikation, eine besondere Aufmerksamkeit zuteilwerden muss. Die Kontaktierung eines Spezialisten kann aber ebenso durch das medizinische Personal erfolgen.

Die vorliegende Erfindung beschreibt Verfahren und Vorrichtungen, die die Abläufe innerhalb einer Dialysestation oder bei der Heimdialyse sicherer, bequemer und einfacher machen. Dem Fachmann ist klar, dass sich die beschriebenen Verfahren nicht nur auf die Dialyse anwenden lassen, sondern überall dort, wo medizintechnische Geräte und betreuendes Personal entfernt voneinander interagieren können.

## Patentansprüche

1. Verfahren zur entfernten Überwachung und Steuerung von medizinischen Fluidmanagementgeräten mit zumindest einer Ein- und Ausgabevorrichtung, vorzugsweise einem Touchscreendisplay, wobei
eine Datenübertragung stattfindet, bei der zumindest Teile des Informationsgehaltes der zumindest einen Ein- und Ausgabevorrichtung eines medizinischen Fluidmanagementgeräts zu zumindest einem entfernten Kontrollgerät übertragen und ausgegeben werden, und wobei Bedienereingaben an zumindest einer Ein- und Ausgabevorrichtung des zumindest einen entfernten Kontrollgeräts, vorzugsweise einem Touchscreen, eingebbar sind, die das medizinischen Fluidmanagementgerät steuern
**dadurch gekennzeichnet,**
**dass** das medizinische Fluidmanagementgerät selbsttätig einen sicheren Zustand durch ein Abklemmen des extrakorporalen Blutkreislaufes vom vaskulären System des Patienten annimmt, wenn ein Verbindungsabbruch zwischen Fluidmanagementgerät und entferntem Kontrollgerät detektiert wird, wobei der Verbindungsabbruch dadurch detektiert wird, dass eine vom entfernten Kontrollgerät bei ordnungsgemäßem Empfang einer Übertragung vom medizinischen Fluidmanagementgerät an das medizinische Fluidmanagementgerät gesendete Bestätigungsmeldung vom medizinischen Fluidmanagementgerät nicht empfangen wird,
oder wenn eine vom entfernten Kontrollgerät bei ordnungsgemäßem Empfang einer Übertragung vom medizinischen Fluidmanagementgerät an das medizinische Fluidmanagementgerät mit einer Zeitangabe gesendete Bestätigungsmeldung vom medizinischen Fluidmanagementgerät gegenüber einem Erwartungszeitpunkt verspätet empfangen wird.

2. Verfahren nach Anspruch 1, **dadurch gekennzeichnet, dass** die Datenübertragung mit einem Zeitstempel versehen wird, der mit übertragen wird.

3. Verfahren nach einem der vorhergehenden Ansprüche, **dadurch gekennzeichnet, dass** das medizinische Fluidmanagementgerät und/oder das entfernte Kontrollgerät mit einer Kamera und einem Lautsprecher und einem Mikrofon ausgestattet sind, wobei zumindest ein Kamerabild und zumindest ein Mikrofonsignal erzeugt werden kann.

4. Verfahren nach einem der vorhergehenden Ansprüche, **dadurch gekennzeichnet, dass** ein Alarmzustand vom medizinischen Fluidmanagementgerät zu einem entfernten Kontrollgerät übertragen wird und zunächst nur bei dem entfernten Kontrollgerät als akustischer, optischer oder haptischer Alarm ausgegeben wird.

5. Verfahren nach einem der vorhergehenden Ansprüche, **dadurch gekennzeichnet, dass** Bedieneingaben an dem entfernten Kontrollgerät eine Wertigkeit erhalten und zum medizinischen Fluidmanagementgerät übertragen werden.

6. Verfahren nach einem der vorhergehenden Ansprüche, **dadurch gekennzeichnet, dass** auf der Ausgabevorrichtung des entfernten Kontrollgeräts ein Lageplan von medizinischen Fluidmanagementgeräten einer Behandlungsstation angezeigt wird.

7. System bestehend aus einem medizinischen Fluidmanagementgerät mit zumindest einer Ein- und Ausgabevorrichtung, vorzugsweise einem Touchscreendisplay und zumindest einem entfernten Kontrollgerät mit zumindest einer Ein- und Ausgabevorrichtung, vorzugsweise einem Touchscreendisplay,
wobei das medizinische Fluidmanagementgerät und das entfernte Kontrollgerät dazu eingerichtet sind, Daten zu übertragen, wobei zumindest Teile des Informationsgehaltes der zumindest einen Ein- und Ausgabevorrichtung des medizinischen Fluidmanagementgeräts zu zumindest einem entfernten Kontrollgerät übertragen werden,
und wobei das entfernte Kontrollgerät dazu eingerichtet ist, den vom medizinischen Fluidmanagementgerät übertragenen Informationsgehalt auf der zumindest einen Ein- und Ausgabevorrichtung anzuzeigen und Bedienereingaben an der zumindest einen Ein- und Ausgabevorrichtung an das medizinische Fluidmanagementgerät zu übertragen und wobei das entfernte Kontrollgerät dazu eingerichtet ist, bei ordnungsgemäßem Empfang einer Übertragung vom medizinischen Fluidmanagementgerät eine Bestätigungsmeldung an das medizinische Fluidmanagementgerät zu senden,
oder wobei das entfernte Kontrollgerät dazu eingerichtet ist, bei ordnungsgemäßem Empfang einer Übertragung vom medizinischen Fluidmanagementgerät eine Bestätigungsmeldung mit einer Zeitangabe an das medizinische Fluidmanagementgerät zu senden,
**dadurch gekennzeichnet,**
**dass** das medizinische Fluidmanagementgerät dazu eingerichtet ist, selbsttätig einen sicheren Zustand durch ein Abklemmen des extrakorporalen Blutkreislaufes vom vaskulären System des Patienten anzunehmen, wenn ein Verbindungsabbruch zwischen Fluidmanagementgerät und entferntem Kontrollgerät detektiert wird, wobei der Verbindungsabbruch dadurch detektiert wird, dass die Bestätigungsmeldung vom medizinischen Fluidmanagementgerät nicht, oder, wenn die Bestätigungsmeldung mit einer Zeitangabe gesendet wurde, gegenüber einem Erwartungszeitpunkt verspätet empfangen wird.

8. System nach Anspruch 7, **dadurch gekennzeichnet, dass** das medizinische Fluidmanagementgerät und das entfernte Kontrollgerät dazu eingerichtet sind, die Datenübertragung mit einem Zeitstempel zu versehen.

9. System nach einem der Ansprüche 7 bis 8, **dadurch gekennzeichnet, dass** das medizinische Fluidmanagementgerät und/oder das entfernte Kontrollgerät mit einer Kamera und einem Lautsprecher und einem Mikrofon ausgestattet sind, wobei zumindest ein Kamerabild und zumindest ein Mikrofonsignal erzeugt werden kann.

10. System nach einem der Ansprüche 7 bis 9, **dadurch gekennzeichnet, dass** das medizinische Fluidmanagementgerät dazu eingerichtet ist, einen Alarmzustand zu einem entfernten Kontrollgerät zu übertragen und die Ausgabe eines akustischen, optischen oder haptischen Alarm zeitweise zu unterdrücken.

11. System nach einem der Ansprüche 7 bis 10, **dadurch gekennzeichnet, dass** das entfernte Kontrollgerät dazu eingerichtet ist, Bedieneingaben mit einer Wertigkeit zu versehen und zu dem medizinischen Fluidmanagementgerät zu übertragen.

12. System nach einem der Ansprüche 7 bis 11, **dadurch gekennzeichnet, dass** das entfernte Kontrollgerät dazu eingerichtet ist, auf der Ausgabevorrichtung des entfernten Kontrollgeräts einen Lageplan von medizinischen Fluidmanagementgeräten in einer Behandlungsstation anzuzeigen.

13. Medizinisches Fluidmanagementgerät ausgeführt mit den auf das medizinische Gerät bezogenen Merkmalen von zumindest einem der Ansprüche 7 bis 12.

## Claims

1. A method for remote monitoring and controlling of medical fluid management devices comprising at least one input and output device, preferably a touchscreen display, wherein
a data transmission takes place, in the case of which at least parts of the information content of the at least one input and output device of a medical fluid management device are transmitted and output to at least one remote control device, and wherein operator inputs, which control the medical fluid management device, can be input on at least one input and output device of the at least one remote control device, preferably a touchscreen,
**characterized in**
**that** the medical fluid management device automatically assumes a secure state by clamping off the extracorporeal blood circulation from the vascular system of the patient when a connection termination between fluid management device and remote control device is detected, wherein the connection termination is detected in that a confirmation message sent by the remote control device to the medical fluid management device upon properly receiving a transmission from the medical fluid management device is not received by the medical fluid management device,
or when a confirmation message sent by the remote control device with a time indication to the medical fluid management device upon properly receiving a transmission from the medical fluid management device is received late by the medical fluid management device compared with an expected time.

2. The method according to claim 1, **characterized in that** the data transmission is provided with a time stamp, which is also transmitted.

3. The method according to one of the preceding claims, **characterized in that** the medical fluid management device and/or the remote control device are equipped with a camera and a loudspeaker and a microphone, wherein at least one camera image and at least one microphone signal can be generated.

4. The method according to one of the preceding claims, **characterized in that** an alarm state is transmitted from the medical fluid management device to a remote control device and is initially output as acoustic, optical or haptic alarm only at the remote control device.

5. The method according to one of the preceding claims, **characterized in that** operator inputs at the remote control device receive a priority and are transmitted to the medical fluid management device.

6. The method according to one of the preceding claims, **characterized in that** a layout of medical fluid management devices of a treatment ward is displayed on the output device of the remote control device.

7. A system consisting of a medical fluid management device comprising at least one input and output device, preferably a touchscreen display, and at least one remote control device comprising at least one input and output device, preferably a touchscreen display,
wherein the medical fluid management device and the remote control device are equipped to transmit data, wherein at least parts of the information content of the at least one input and output device of the medical fluid management device are transmitted to at least one remote control device,
and wherein the remote control device is equipped to display the information content transmitted from the medical fluid management device on the at least one input and output device, and to transmit operator input on the at least one input and output device to the medical fluid management device, and wherein the remote control device is equipped to transmit a confirmation message to the medical fluid management device upon properly receiving a transmission from the medical fluid management device,
or wherein the remote control device is equipped to send a confirmation message with a time indication to the medical fluid management device upon properly receiving a transmission from the medical fluid management device,
**characterized in**
**that** the medical fluid management device is equipped to automatically assume a secure state by clamping off the extracorporeal blood circulation from the vascular system of the patient when a connection termination between fluid management device and remote control device is detected, wherein the connection termination is detected in that the confirmation message is not received by the medical fluid management device or, when the confirmation message was sent with a time indication, is received late compared with an expected time.

8. The system according to claim 7, **characterized in that** the medical fluid management device and the remote control device are equipped to provide the data transmission with a time stamp.

9. The system according to one of claims 7 to 8, **characterized in that** the medical fluid management device and/or the remote control device are equipped with a camera and a loudspeaker and a microphone, wherein at least one camera image and at least one microphone signal can be generated.

10. The system according to one of claims 7 to 9, **characterized in that** the medical fluid management device is equipped to transmit an alarm state to a remote control device and to temporarily suppress the output of an acoustic, optical or haptic alarm.

11. The system according to one of claims 7 to 10, **characterized in that** the remote control device is equipped to provide a priority to operator inputs and to transmit them to the medical fluid management device.

12. The system according to one of claims 7 to 11, **characterized in that** the remote control device is equipped to display a layout of medical fluid management devices in a treatment ward on the output device of the remote control device.

13. A medical fluid management device embodied with the features of at least one of claims 7 to 12 relating to the medical device.

## Revendications

1. Procédé de surveillance et de commande distantes d'appareil médicaux de gestion de fluide comportant au moins un dispositif de saisie et d'édition, de préférence un afficheur à écran tactile, dans lequel
il y a un transfert de données lors duquel au moins des parties de la teneur informative de l'au moins un dispositif de saisie et d'édition d'un appareil médical de gestion de fluide sont transférées à au moins un appareil de commande distant et éditées et des saisies d'utilisateur faites sur au moins un dispositif de saisie et d'édition de l'au moins un appareil de commande distant, de préférence un écran tactile, peuvent être entrées, lesquelles saisies commandent l'appareil médical de gestion de fluide,
**caractérisé en ce que**
l'appareil médical de gestion de fluide prend automatiquement un état de sécurité par un débranchement du circuit sanguin extracorporel du système vasculaire du patient lorsqu'une interruption de la liaison entre l'appareil de gestion de fluide et l'appareil de commande distant est détectée, l'interruption de la liaison étant détectée du fait qu'un message de confirmation envoyé à l'appareil médical de gestion de fluide par l'appareil de commande distant, en cas de réception correcte d'une transmission de l'appareil de gestion de fluide, n'est pas reçu par l'appareil de gestion de fluide,
ou lorsqu'un message de confirmation envoyé avec une indication horaire à l'appareil de gestion de fluide par l'appareil de commande distant, en cas de réception correcte d'une transmission depuis l'appareil médical de gestion de fluide, est reçu en retard par rapport à un moment où on l'attendait.

2. Procédé selon la revendication 1, **caractérisé en ce que** la transmission de données est pourvue d'un horodatage qui est transmis en même temps.

3. Procédé selon une des revendications précédentes, **caractérisé en ce que** l'appareil médical de gestion de fluide et/ou l'appareil de commande distant sont équipés d'une caméra et d'un haut-parleur et d'un micro, au moins une image de caméra et au moins un signal de micro pouvant être générés.

4. Procédé selon une des revendications précédentes, **caractérisé en ce qu'**un état d'alarme est transmis par l'appareil médical de gestion de fluide à un appareil de commande distant et n'est ensuite édité sous forme d'une alarme acoustique, optique ou tactile que lorsque l'appareil de commande est distant.

5. Procédé selon une des revendications précédentes, **caractérisé en ce que** les saisies d'utilisateur sur l'appareil de commande distant se voient attribuer une valeur et sont transmises à l'appareil médical de gestion de fluide.

6. Procédé selon une des revendications précédentes, **caractérisé en ce que**, sur le dispositif d'édition de l'appareil de commande distant, un plan de situation des appareils médicaux de gestion de fluide d'une station de traitement est affiché.

7. Système composé d'un appareil médical de gestion de fluide, comportant au moins un dispositif de saisie et d'édition, de préférence un afficheur à écran tactile et au moins un appareil de commande distant comportant au moins un dispositif de saisie et d'édition, de préférence un afficheur à écran tactile,
dans lequel l'appareil médical de gestion de fluide et l'appareil de commande distant sont conçus pour transmettre des données, au moins des parties de la teneur informative de l'au moins un dispositif de saisie et d'édition de l'appareil médical de gestion de fluide étant transmises à au moins un appareil de commande distant,
et l'appareil de commande distant étant conçu pour afficher la teneur informative transmise par l'appareil médical de gestion de fluide sur l'au moins un dispositif de saisie et d'édition et transmettre les saisies d'utilisateur faites sur l'au moins un dispositif de saisie et d'édition à l'appareil médical de gestion de fluide et l'appareil de commande distant étant conçu pour, en cas de réception correcte d'une transmission de l'appareil médical de gestion de fluide, envoyer un message de confirmation à l'appareil médical de gestion de fluide,
ou l'appareil de commande distant étant conçu pour, en cas de réception correcte d'une transmission de l'appareil médical de gestion de fluide, envoyer un message de confirmation comportant une indication horaire à l'appareil médical de gestion de fluide,
**caractérisé en ce que**
l'appareil médical de gestion de fluide est conçu pour prendre automatiquement un état de sécurité par un débranchement du circuit sanguin extracorporel du système vasculaire du patient lorsqu'une interruption de la liaison entre l'appareil de gestion de fluide et l'appareil de commande distante est détectée, l'interruption de la liaison étant détectée du fait que le message de confirmation n'est pas reçu par l'appareil médical de gestion de fluide ou, si le message de confirmation a été envoyé avec une indication horaire, est reçu en retard par rapport à un moment où on l'attendait.

8. Système selon la revendication 7, **caractérisé en ce que** l'appareil médical de gestion de fluide et l'appareil de commande distant sont conçus pour pourvoir la transmission de données d'un horodatage.

9. Système selon une des revendications 7 à 8, **caractérisé en ce que** l'appareil médical de gestion de fluide et/ou l'appareil de commande distant sont équipés d'une caméra et d'un haut-parleur et d'un micro, au moins une image de caméra et au moins un signal de micro pouvant être générés.

10. Système selon une des revendications 7 à 9, **caractérisé en ce que** l'appareil médical de gestion de fluide est conçu pour transmettre un état d'alarme à un appareil de commande distant et pour arrêter temporairement l'émission d'une alarme acoustique, optique ou tactile.

11. Système selon une des revendications 7 à 10, **caractérisé en ce que** l'appareil de commande distant est conçu pour attribuer une valeur aux saisies d'utilisateur et les transmettre à l'appareil médical de gestion de fluide.

12. Système selon une des revendications 7 à 11, **caractérisé en ce que** l'appareil de commande distant est conçu pour afficher, sur le dispositif d'édition de l'appareil de commande distant, un plan de situation des appareils médicaux de gestion de fluide dans une station de traitement.

13. Appareil médical de gestion de fluide réalisé avec les caractéristiques se rapportant à l'appareil médical d'au moins une des revendications 7 à 12.
